# EUROPEAN PATENT SPECIFICATION

(11) **EP 2 442 841 B1**
(45) Date of publication and mention of the grant of the patent: **13.11.2013**
(21) Application number: 09791759.5
(22) Date of filing: 20.08.2009
(51) Int. Cl.: A61L 31/10, A61L 31/14, A61L 31/16

(54) **IMPLANTABLE MEDICAL DEVICES AND COATINGS THEREFOR COMPRISING BLOCK COPOLYMERS OF POLY (ETHYLENE GLYCOL) AND A POLY (LACTIDE-GLYCOLIDE)**
IMPLANTIERBARE MEDIZINISCHE VORRICHTUNGEN UND BESCHICHTUNGEN DAFÜR MIT BLOCKCOPOLYMEREN AUS POLY (ETHYLENGLYCOL) UND POLY (LACTID-GLYCOLID)
DISPOSITIFS MÉDICAUX IMPLANTABLES ET REVÊTEMENTS POUR CEUX-CI COMPRENANT DES COPOLYMÈRES SÉQUENCÉS DE POLY(ÉTHYLÈNE GLYCOL) ET D'UN POLY(LACTIDE-GLYCOLIDE)

(30) Priority: 15.06.2009 US 484951
(43) Date of publication of application: 25.04.2012
(73) Proprietor: Abbott Cardiovascular Systems Inc., Santa Clara, CA 95054-2807 (US)
(72) Inventor: LIM, Florencia, Union City, CA 94587 (US); TROLLSAS, Mikael, O., San Jose, CA 95124 (US); NGO, Michael, Huy, San Jose, CA 95128 (US); HU, Jie, Sunnyvale, CA 94087 (US); HOSSAINY, Syed, F., A., Fremont, CA 94555 (US); SHERMAN, David, J., Tarzana, CA 91356 (US)
(74) Representative: Barlocci, Anna
(86) International application number: PCT/US2009/054533
(87) International publication number: WO 2010/147603

(56) References cited:
- EP-A1- 1 932 551
- EP-A2- 1 891 993
- WO-A2-2008/121508
- US-A1- 2004 106 987

## Description

### FIELD OF THE INVENTION

This invention relates to the fields of organic chemistry, polymer chemistry, materials science and medical devices.

### BACKGROUND OF THE INVENTION

The discussion that follows is intended solely as background information to assist in the understanding of the invention herein; nothing in this section is intended to be, nor is it to be construed as, prior art to this invention.

Until the mid-1980s, the accepted treatment for atherosclerosis, i.e., narrowing of the coronary artery(ies) was coronary by-pass surgery. While effective and evolved to a relatively high degree of safety for such an invasive procedure, by-pass surgery still involves potentially serious complications, and in the best of cases, an extended recovery period.

With the advent of percutaneous transluminal coronary angioplasty (PTCA) in 1977, the scene changed dramatically. Using catheter techniques originally developed for heart exploration, inflatable balloons were employed to re-open occluded regions in arteries. The procedure was relatively non-invasive, took a very short time compared to by-pass surgery and the recovery time was minimal. However, PTCA brought with it another problem, elastic recoil of the stretched arterial wall which could undo much of what was accomplished and, in addition, PTCA failed to satisfactorily ameliorate another problem, restenosis, the re-clogging of the treated artery.

The next improvement, advanced in the mid-1980s, was use of a stent to hold the vessel walls open after PTCA. This for all intents and purposes put an end to elastic recoil but did not entirely resolve the issue of restenosis. That is, prior to the introduction of stents, restenosis occurred in 30 - 50% of patients undergoing PTCA. Stenting reduced this to about 15 - 30%, much improved but still more than desirable.

In 2003, the drug-eluting stent (DES) was introduced. The drugs initially employed with the DES were cytostatic compounds, compounds that curtailed the proliferation of cells that contributed to restenosis. As a result, restenosis was reduced to about 5 - 7%, a relatively acceptable figure. Today, the DES is the default industry standard for the treatment of atherosclerosis and is rapidly gaining favor for treatment of stenoses of blood vessels other than coronary arteries such as peripheral angioplasty of the femoral artery.

One of the key issues with DESs is control of the rate of release of the drug from the coating. If the bulk of the drug is released soon after implantation, known in the art as "burst release," the intent of providing prolonged delivery is defeated. Furthermore, burst release may result in local drug concentrations that are toxic. On the other hand, drug delivery release rates which are too slow may not provide a sufficiently high local concentration to have the intended therapeutic effect. Control of drug release must be balanced with maintaining an acceptable mechanical integrity of the coating, particularly after sterilization.

EP 1932551 discloses an implantable device having a coating made of a polymer comprising poly(lactic acid) or block-copolymers thereof, including diblock or triblockcopolymers having at least one biocompatible moiety including poly(ethylene glycol) or glycolide.

EP 1891993 discloses an implant made of a biocorrodible metallic material, the implant comprising a coating or cavity filling comprising a diblock or triblock copolymer made of (i) a poly(D,L-lactide-co-glycolide) block and (ii) a polyethylene glycol block as well as a method for its preparation.

WO 2008/121508 discloses an implantable medical device comprising a structural element fabricated from a polymeric material, which includes a block copolymer having a continuous phase block that is immiscible with a discrete phase block, wherein the block copolymer may comprise a di-block copolymer or a tri-block copolymer.

US 2004/106987 discloses a medical device comprising (a) a therapeutic agent containing layer; and (b) a polymeric covering layer disposed over the therapeutic-agent layer, which comprises one or more polymers selected from (i) a block copolymer comprising at least two polymeric blocks A and B, wherein A is a polyolefin block and wherein B is a vinyl aromatic block, (ii) a polymer or copolymer of lactic acid, (iii) a polymer or copolymer of glycolic acid, and (iv) a tyrosine-based polymer or copolymer. It also discloses a medical device which comprises (a) polymeric layer comprising a removable component as well as (i) a block copolymer comprising at least two polymeric blocks A and B, wherein A is a polyolefin block and B is a vinyl aromatic block and/or (ii) a tyrosine-based polymer or copolymer; and (b) a high-molecular-weight therapeutic agent disposed below or within the polymeric layer.

Coatings for DES that both control drug release and exhibit good mechanical properties are needed. The present invention provides such coatings.

### SUMMARY OF THE INVENTION

The current invention is directed to implantable medical devices and coatings thereon and methods of treatment using such devices.

Thus, in one aspect the current invention is an implantable medical device comprising:
a device body;
a coating disposed over at least a portion of the outer surface of the device body, the coating comprising;
   a polymer selected from the group consisting of a semi-crystalline A-B block copolymer, and a semi-crystalline A-B-A block copolymer:
   wherein B is a poly(ethylene glycol) block with a weight average molecular weight of 1000 to 30000 Daltons, and
   A is formed from monomers comprising glycolide; and one or more monomers selected from the group consisting of L-lactide, D-lactide, meso-lactide, and combinations thereof;
      wherein the molar concentration of ethylene glycol in the polymer is 1% to 20% and the molar concentration of the sum of L-lactide, D-lactide, and meso-lactide in the A block is 70% to 95%; and
      wherein the weight average molecular weight of the polymer is not less than 50,000 Daltons and not more than 1,000,000 Daltons; and a drug, and wherein the mass ratio of drug to polymer is less than 1, as mentioned in claim 1.

In an aspect of the present invention, the B block of the A-B block copolymer or A-B-A block copolymer has a weight average molecular weight of 1000 to 20000 Daltons.

In an aspect of the present invention, the B block of the A-B block copolymer or A-B-A block copolymer has a weight average molecular weight of 1000 to 10000 Daltons.

In an aspect of the present invention, the molar concentration of ethylene glycol is 1% to 10% in the A-B block copolymer or the A-B-A block copolymer.

In an aspect of the present invention, the molar concentration of the sum of L-lactide, D-lactide, and meso-lactide in the A block is 80% to 95%.

In an aspect of the present invention, the molar concentration of the sum of L-lactide, D-lactide, and meso-lactide in the A block is 82% to 95%.

In an aspect of the present invention, the device is a stent.

In an aspect of the present invention, the stent is biodegradable, resorbable, or a combination thereof.

In an aspect of the present invention, the stent body comprises poly(L-lactide).

In an aspect of the present invention, the drug is selected from the group consisting of paclitaxel, docetaxel, estradiol, 17-beta-estradiol, nitric oxide donors, super oxide dismutases, super oxide dismutases mimics, 4-amino-2,2,6,6-tetramethylpiperidine-1-oxyl (4-amino-TEMPO), rapamycin (sirolimus), Biolimus A9 (Biosensors International, Singapore), deforolimus, AP23572 (Ariad Pharmaceuticals), tacrolimus, temsirolimus, pimecrolimus, novolimus, zotarolimus (ABT-578), 40-O-(2-hydroxy)ethyl-rapamycin (everolimus), 40-O-(3-hydroxypropyl), 40-O-[2-(2-hydroxy)ethoxy]ethyl-rapamycin, 40-O-tetrazolylrapamycin, 40-epi-(N1-tetrazolyl)-rapamycin, dexamethasone, dexamethasone acetate, dexamethasone derivatives, γ-hiridun, clobetasol, pimecrolimus, imatinib mesylate, midostaurin, feno fibrate, and any combination thereof.

In an aspect of the present invention, the drug is selected from the group consisting of rapamycin (sirolimus), Biolimus A9 (Biosensors International, Singapore), deforolimus, AP23572 (Ariad Pharmaceuticals), tacrolimus, temsirolimus, pimecrolimus, novolimus, zotarolimus (ABT-578), 40-O-(2-hydroxy)ethyl-rapamycin (everolimus), 40-O-(3-hydroxypropyl), 40-O-[2-(2-hydroxy)ethoxy]ethyl-rapamycin, 40-O-tetrazolylrapamycin, 40-epi-(N1-tetrazolyl)-rapamycin, dexamethasone, dexamethasone acetate, dexamethasone derivatives, and any combination thereof.

In an aspect of the present invention, the drug is everolimus, zotarolimus, or a combination thereof.

In an aspect of the present invention, the polymer is an A-B block copolymer.

In an aspect of the present invention, the polymer is an A-B-A block copolymer.

In an aspect of the present invention, the polymer is selected from the group consisting of a polymer having about 85 mol % L-lactide, D-lactide, or a combination thereof in the A-block where the L-lactide and/or D-lactide are among the monomers used in forming the A block, and about 1 mol % ethylene glycol in the polymer where the B block is polyethylene glycol with a weight average molecular weight of about 6000, a polymer having about 85 mol % L-lactide, D-lactide, or combination thereof in the A block where the L-lactide and/or D-lactide are among the monomers used in forming the A block, and about 4 mol % ethylene glycol in the polymer where the B block is polyethylene glycol with a weight average molecular weight of about 6000, and a polymer having about 85 mol % L-lactide, D-lactide, or a combination thereof in the A-block where the L-lactide, and/or D-lactide are among the monomers used in forming the A block, and about 5 mol % ethylene glycol in the polymer, where the B block is polyethylene glycol with a weight average molecular weight of about 5000.

In an aspect of the present invention, the polymer is selected from the group consisting of a polymer having about 85 mol % L-lactide, D-lactide, or a combination thereof in the polymer where the L-lactide and/or D-lactide are among the monomers used in forming the A block, and about 1 mol % ethylene glycol in the polymer where the B block is polyethylene glycol with a weight average molecular weight of about 6000, a polymer having about 85 mol % L-lactide, D-lactide, or combination thereof in the polymer where the L-lactide and/or D-lactide are among the monomers used in forming the A block, and about 4 mol % ethylene glycol in the polymer where the B block is polyethylene glycol with a weight average molecular weight of about 6000, and a polymer having about 85 mol % L-lactide, D-lactide, or a combination thereof in the polymer where the L-lactide, and/or D-lactide are among the monomers used in forming the A block, and about 5 mol % ethylene glycol in the polymer, where the B block is polyethylene glycol with a weight average molecular weight of about 5000.

In an aspect of the present invention, the drug to polymer ratio is 0.75 or less than 0.75.

In an aspect of the present invention, the drug to polymer ratio is 0.5 or less than 0.5.

In an aspect of the present invention, the device is a stent, the drug to polymer ratio is 0.5 or less than 0.5, and the drug is everolimus or zotarolimus.

In an aspect of the present invention, the device exhibits a cumulative drug at 24 hours of not greater than 60%.

In an aspect of the present invention, the device exhibits a cumulative drug release at 72 hours of not greater than 90%.

In an aspect of the present invention, the device exhibits a cumulative drug release at 72 hours of not greater than 75%.

Thus, another aspect the current invention relates to an implantable medical device comprising an implantable medical device comprising:
a device body;
a coating formed by:
   disposing over at least a portion of the outer surface of the device body one or more coating solutions, at least one coating solution comprising:
      a polymer selected from the group consisting of a semi-crystalline A-B block copolymer, and a semi-crystalline A-B-A block copolymer:
         wherein B is a poly(ethylene glycol) block with a weight average molecular weight of 1000 to 30000 Daltons, and A is formed from monomers comprising glycolide, and one or more monomers selected from the group consisting of L-lactide, D-lactide, meso-lactide, and combinations thereof; and
         wherein the molar concentration of ethylene glycol in the polymer is 1% to 20% and the molar concentration of the sum of L-lactide, D-lactide, and meso-lactide in the A block is 70% to 95%; and wherein the weight average molecular weight of the polymer is not less than 50,000 Daltons and not more than 1,000,000 Daltons;
a drug; and
a solvent;
   wherein the mass ratio of drug to polymer in the coating solution is less than 1,
and removing the solvent.
In an aspect of the present invention, the device is a stent;
the polymer is selected from the group consisting of a polymer having about 85 mol % L-lactide, D-lactide, or a combination thereof in the A-block where the L-lactide and/or D-lactide are among the monomers used in forming the A block, and about 1 mol % ethylene glycol in the polymer where the B block is polyethylene glycol with a weight average molecular weight of about 6000, a polymer having about 85 mol % L-lactide, D-lactide, or combination thereof in the A block where the L-lactide and/or D-lactide are among the monomers used in forming the A block, and about 4 mol% ethylene glycol in the polymer where the B block is polyethylene glycol with a weight average molecular weight of about 6000, and a polymer having about 85 mol % L-lactide, D-lactide, or a combination thereof in the A-block where the L-lactide, and/or D-lactide are among the monomers used in forming the A block, and about 5 mol % ethylene glycol in the polymer, where the B block is polyethylene glycol with a weight average molecular weight of about 5000;
the drug is selected from the group consisting of paclitaxel, docetaxel, estradiol, 17-beta-estradiol, nitric oxide donors, super oxide dismutases, super oxide dismutases mimics, 4-amino-2,2,6,6-tetramethylpiperidine-1-oxyl (4-amino-TEMPO), rapamycin (sirolimus), Biolimus A9 (Biosensors International, Singapore), deforolimus, AP23572 (Ariad Pharmaceuticals), tacrolimus, temsirolimus, pimecrolimus, novolimus, zotarolimus (ABT-578), 40-O-(2-hydroxy)ethyl-rapamycin (everolimus), 40-O-(3-hydroxypropyl), 40-O-[2-(2-hydroxy)ethoxy]ethyl-rapamycin, 40-O-tetrazolylrapamycin, 40-epi-(N1-tetrazolyl)-rapamycin, dexamethasone, dexamethasone acetate, dexamethasone derivatives, γ-hiridun, clobetasol, pimecrolimus, imatinib mesylate, midostaurin, feno fibrate, and any combination thereof;
and the drug to polymer ratio is from about 2:3 to about 1:3.

In an aspect of the present invention, a method for the treatment of a disease or condition comprising implanting in a patient in need thereof an implantable medical device as described above.

In an aspect of the present invention, the disease or condition is selected from the group consisting of coronary artery disease (CAD), peripheral vascular disease (PVD), restenosis, atherosclerosis, thrombosis, hemorrhage, vascular dissection or perforation, vascular aneurysm, vulnerable plaque, chronic total occlusion, claudication, anastomotic proliferation (for vein and artificial grafts), bile duct obstruction, urethral obstruction, tumor obstruction, and combinations of these.

### DETAILED DESCRIPTION

Use of the singular herein includes the plural and vice versa unless expressly stated to be otherwise. That is, "a" and "the" refer to one or more of whatever the word modifies. For example, "a drug" may refer to one drug, two drugs, etc. Likewise, "the polymer" may mean one polymer or a plurality of polymers. By the same token, words such as, without limitation, "drugs" and "polymers" would refer to one drug or polymer as well as to a plurality of drugs or polymers unless it is expressly stated or obvious from the context that such is not intended.

As used herein, unless specified otherwise, any words of approximation such as without limitation, "about," "essentially," "substantially" and the like mean that the element so modified need not be exactly what is described but can vary from the description by as much as ±15% without exceeding the scope of this invention.

As used herein, any ranges presented are inclusive of the end-points. For example, "a temperature between 10 °C and 30 °C" or "a temperature from 10 °C to 30 °C" includes 10 °C and 30 °C, as well as any temperature in between.

As used herein, the use of "preferred," "preferably," "more preferred," and the like to modify an aspect of the invention refers to preferences as they existed at the time of filing of the patent application.

"Physiological conditions" refer to conditions to which an implant is exposed within the body of an animal (e.g., a human). Physiological conditions include, but are not limited to, "normal" body temperature for that species of animal (approximately 37°C for a human) and an aqueous environment of physiologic ionic strength, pH and enzymes. In some cases, the body temperature of a particular animal may be above or below what would be considered "normal" body temperature for that species of animal. For example, the body temperature of a human may be above or below approximately 37°C in certain cases depending on the ailment from which the human is suffering. The scope of the present invention encompasses such cases where the physiological conditions (e.g., body temperature) of an animal are not considered "normal."

As used herein, a "polymer" refers to a molecule comprised of repeating "constitutional units." The constitutional units derive from the reaction of monomers. As a non-limiting example, ethylene (CH₂=CH₂) is a monomer that can be polymerized to form polyethylene, CH₃CH₂(CH₂CH₂)ₙCH₂CH₃, wherein the constitutional unit is -CH₂CH₂-, ethylene having lost the double bond as the result of the polymerization reaction. A polymer may be derived from the polymerization of several different monomers and therefore may comprise several different constitutional units. Such polymers are referred to as "copolymers." The constitutional units themselves can be the product of the reactions of other compounds. Those skilled in the art, given a particular polymer, will readily recognize the constitutional units of that polymer and will equally readily recognize the structure of the monomer from which the constitutional units derive. Polymers may be straight or branched chain, star-like or dendritic, or one polymer may be attached (grafted) onto another. Polymers may have a random disposition of constitutional units along the chain, the constitutional units may be present as discrete blocks, or constitutional units may be so disposed as to form gradients of concentration along the polymer chain. Polymers may be cross-linked to form a network.

As used herein, a "block copolymer" refers to a copolymer where instead of the different types of constitutional units having a random distribution along the polymer chain, the constitutional units are arranged as discrete "blocks" or "segments." Block copolymers may be regular or random block copolymers. A regular block copolymer has, for example and without limitation, the general structure: ...x-x-x-y-y-y-z-z-z-x-x-x..., while a random block polymer has, for example and without limitation, the general structure: ...x-x-x-z-z-x-x-y-y-y-y-z-z-z-x-x-z-z-z-.... The blocks may be homopolymers, that is blocks of one type of constitutional unit, or the block may include more than one type of constitutional unit. The arrangement of the constitutional units within a block may also be random or regular.

As used herein, a "polymer segment" or "polymer block" refers to a polymeric species that forms part of a larger polymer. For the purposes of this invention, the polymer segments or blocks are also polymers; thus they are referred to herein as "polymer segments", "polymer blocks", or sometime simply "segments" or "blocks." The terms are used interchangeably.

As used herein, when reference is made to a polymer having X mol% of a particular monomer such refers to the mole percent of the monomer used to form the polymer.

As used herein, the term "semi-crystalline" refers to polymers having crystalline domain(s)/region(s) and amorphous domain(s)/region(s).

As used herein, "biocompatible" refers to a polymer or other material that both in its intact, that is, as synthesized, state and in its decomposed state, i.e., its degradation products, is not, or at least is minimally, toxic to living tissue; does not, or at least minimally and reparably, injure(s) living tissue; and/or does not, or at least minimally and/or controllably, cause(s) an immunological reaction in living tissue.

As used herein, the terms "biodegradable", "bioerodable", "degraded," and "eroded," are used interchangeably, and refer to polymers, coatings, coating layers, and other materials that are capable of being completely or substantially completely, chemically or biochemically decomposed over time when exposed to physiological conditions, and can be degraded into fragments that can pass through the kidney membrane of an animal. Smaller fragments may be resorbable.

As used herein, the term "resorbable" refers to materials such as, without limitation, polymers, coatings, and coating layers, that are capable of being completely, or substantially completely, dissolved and/or absorbed over time when exposed to physiological conditions, and subsequently eliminated by the body. Materials that are resorbable do not chemically or biochemically degrade into smaller fragments when exposed to physiological conditions.

For coatings on implantable medical devices, or polymers forming such coatings, it is understood that after the process of degradation or resorption has been completed or substantially completed, the device will be free of, or substantially free of, the coating or polymer. In some embodiments, a negligible residue may be left behind.

Conversely, "biostable" refers to materials that are not biodegradable or resorbable.

As used herein, an "implantable medical device" refers to any type of appliance that is totally or partly introduced, surgically or medically, into a patient's body or by medical intervention into a natural orifice, and which is intended to remain there after the procedure. The duration of implantation may be essentially permanent, i.e., intended to remain in place for the remaining lifespan of the patient; may be until the device biodegrades; or may be until it is physically removed. Examples of implantable medical devices include, without limitation, implantable cardiac pacemakers and defibrillators; leads and electrodes for the preceding; implantable organ stimulators such as nerve, bladder, sphincter and diaphragm stimulators, cochlear implants; prostheses, vascular grafts, self-expandable stents, balloon-expandable stents, stent-grafts, grafts, artificial heart valves, foramen ovale closure devices, cerebrospinal fluid shunts, and intrauterine devices. An implantable medical device specifically designed and intended solely for the localized delivery of a drug is within the scope of this invention. Implantable medical devices can be made of virtually any material including metals and/or polymers, where polymers includes biostable polymers, biodegradable polymers, resorbable polymers and any combination of these types of polymers.

One form of implantable medical device is a "stent." A stent refers generally to any device used to hold tissue in place in a patient's body. Particularly useful stents, however, are those used for the maintenance of the patency of a vessel in a patient's body when the vessel is narrowed or closed due to diseases or disorders includirrg, without limitation, tumors (in, for example, bile ducts, the esophagus, the trachea/bronchi, etc.), benign pancreatic disease, coronary artery disease such as, without limitation, atherosclerosis, carotid artery disease, peripheral arterial disease, restenosis and vulnerable plaque.

In the context of a stent, "delivery" refers to introducing and transporting the stent through a bodily lumen to a region, such as a lesion, in a vessel that requires treatment. "Deployment" corresponds to the expansion of the stent within the lumen at the treatment region. Delivery and deployment of a stent are typically accomplished by placing the stent at one end of a catheter, inserting the catheter into a bodily lumen, advancing the catheter to a desired treatment location, expanding the stent at the treatment location, and removing the catheter from the lumen.

As used herein an implantable medical device "device body" refers to a device in a fully formed utilitarian state with an outer surface to which no coating or layer of material different from that of which the device itself is manufactured has been applied. By "outer surface" is meant any surface however spatially oriented that is in contact with bodily tissue or fluids. A common example of a "device body" is a BMS, i.e., a bare metal stent, which, as the name implies, is a fully-formed usable stent that has not been coated with a layer of any material different from the metal of which it is made on any surface that is in contact with bodily tissue or fluids. Of course, device body refers not only to BMSs but to any uncoated device regardless of what it is made of.

As used herein, a material that is described as a layer, a film, or a coating "disposed over" an indicated substrate refers to disposition of the material directly or indirectly over at least a portion of the surface of the substrate. "Directly deposited" means that the material is applied directly onto the surface of the substrate. "Indirectly deposited" means that the material is applied to an intervening layer that has been deposited directly or indirectly over the substrate. The terms "layer", and "coating layer" will be used interchangeably and refer to a layer or film, as described in this paragraph. A coating may comprise one or more layers. Unless the context clearly indicates otherwise, a reference to a coating, layer, or coating layer refers to a layer of material that covers all, or substantially all, of a surface, whether deposited directly or indirectly.

As used herein, "solvent" is defined as a fluid capable of dissolving, partially dissolving, dispersing, or suspending one or more substances to form a uniform dispersion and/or solution, with or without agitation, at a selected temperature and pressure. The fluid may be liquid, gaseous or in a supercritical state. A solvent herein may be a blend of two or more such fluids. As used herein, an "organic solvent" is a fluid the chemical composition of which includes carbon atom(s).

As used herein, a "coating solution" refers to a composition, typically one or more substances combined with a solvent that can be disposed over a substrate, such as an implantable medical device, by a common technique, such as spraying or dipping to deposit the substances on the substrate. The substances may be dissolved, dispersed, or suspended in the solvent.

As used herein, a "coating formulation" refers to the substance or mixture of substances that are disposed over a substrate. If a coating solution is disposed over a substrate with removal of the solvent, the solvent is not part of the "coating formulation" even though the layer deposited may contain residual solvent.

As used herein, a "primer layer" refers to a coating consisting of a material such as, without limitation, a polymer that exhibits good adhesion characteristics to the material of which the substrate is manufactured and also good adhesion characteristics to whatever other material is to be coated on the substrate. Thus, a primer layer serves as an adhesive intermediary layer between a substrate and materials to be carried by the substrate and is, therefore, applied directly to the substrate. Preferred substrates are medical device bodies.

As used herein, a "drug" refers to any substance that, when administered in a therapeutically effective amount to a patient suffering from a disease or condition, has a therapeutic beneficial effect on the health and well-being of the patient. A therapeutic beneficial effect on the health and well-being of a patient includes, but it not limited to: (1) curing the disease or condition; (2) slowing the progress of the disease or condition; (3) causing the disease or condition to retrogress; or, (4) alleviating one or more symptoms of the disease or condition.

As used herein, a drug also includes any substance that when administered to a patient, known or suspected of being particularly susceptible to a disease, in a prophylactically effective amount, has a prophylactic beneficial effect on the health and well-being of the patient. A prophylactic beneficial effect on the health and well-being of a patient includes, but is not limited to: (1) preventing or delaying onset of the disease or condition in the first place; (2) maintaining a disease or condition at a retrogressed level once such level has been achieved by a therapeutically effective amount of a substance, which may be the same as or different from the substance used in a prophylactically effective amount; or, (3) preventing or delaying recurrence of the disease or condition after a course of treatment with a therapeutically effective amount of a substance, which may be the same as or different from the substance used in a prophylactically effective amount, has concluded.

As used herein, "drug" also refers to pharmaceutically acceptable, pharmacologically active derivatives of those drugs specifically mentioned herein, including, but not limited to, salts, esters, amides, and the like. Substances useful for diagnostics are also encompassed by the term "drug" as used herein.

The terms "drug," "bioactive agent", "biologically active agent," "biological agent," "active ingredient," and "therapeutic agent" are used interchangeably herein.

"Prohealing" refers to a drug or agent that promotes or enhances reendothelialization of arterial lumen to expedite healing of the vascular tissue.

As used herein, a "co-drug" is a drug that is administered concurrently or sequentially with another drug to achieve a particular pharmacological effect. The effect may be general or specific. The co-drug may exert an effect different from that of the other drug, or it may promote, enhance or potentiate the effect of the other drug.

As used herein, the term "prodrug" refers to an agent rendered less active by a chemical or biological moiety, which metabolizes into or undergoes *in vivo* hydrolysis to form a drug or an active ingredient thereof. The term "prodrug" can be used interchangeably with terms such as "proagent", "latentiated drugs", and "bioreversible derivatives." Prodrugs can generally be defined as pharmacologically less active chemical derivatives that can be converted *in vivo,* enzymatically or nonenzymatically, to the active, or more active, drug molecules that exert a therapeutic, prophylactic or diagnostic effect.

As used herein, "release rate" refers to the speed of drug release from a drug delivery system per unit of time, for example without limitation 0.1 mg per hour (0.1 mg/hr) or 100 mg per day.

As used herein, a coating, coating layer, or device that "controls the release" of a drug refers to one for which the cumulative release of the drug is less than 90% in 24 hours, but is at least 5% in 72 hours.

As used herein, "cumulative drug release" refers to the total amount of drug released from the drug delivery system up to a given point in time, such as, without limitation, 24 hours. The "cumulative drug release" is usually expressed as a percent of the total drug content of the drug delivery system. In such a calculation, the total drug content that is used in the denominator may be obtained from actual measurements based on percent drug as determined by analytical assay.

As used herein, "release duration," refers to the total time over which a drug is released in a therapeutically effective amount from a drug delivery system or formulation. Thus, for example without limitation, a drug release range of, say, 1 hour to 72 hours means that a therapeutically effective amount of the drug is released over that time period.

As used herein, any measurement of drug release, for example without limitation, release rate or release duration, refers to an in-vitro measurement using a United States Pharmacopeia Type VII apparatus, using porcine serum at a temperature of 37 °C, and optionally with sodium azide added (for example, without limitation, at about 0.1 % w/v).

The present invention provides a block copolymer comprising a poly(ethylene glycol) (PEG) block and at least one polyester block. The block copolymers are useful as coatings on implantable medical devices, or for fabricating implantable medical devices. The polyester block is hydrophobic, imparting hydrophobicity to the block copolymer; and the PEG block is hydrophilic, imparting hydrophilicity to the block copolymer. The block copolymer generally has a weight-average molecular weight (M_{w}) of 50,000 Daltons or higher, preferably 60,000 Daltons or higher, and more preferably, 100,000 Daltons or higher. The M_{w} of the block copolymer is also not more than 1,000,000 Daltons, and preferably not more than 600,000 Daltons.

The polyester block can include any monomers capable of forming ester linkages. In some embodiments, the polyester block can be formed from monomers such as lactide, glycolide, caprolactone, trimethylene carbonate (TMC), or combinations thereof. The polyester block can have various molar concentrations of any of these monomers. For example, the polyester block can have lactide with a molar concentration of at least 60%, or at least 80%. In some embodiments, the polyester block can have glycolide with a molar concentration of between 10% and 75%.

Selection of different monomers for the polyester block allows the design of the molecular structure of the blocks such that the drug/polymer interaction may be optimized to provide for better control of drug release. For example, to provide a controlled release of everolimus from a coating formed of a polyester including poly(L-lactide) (PLLA) and/or poly(L-lactide-co-glycolide) (PLGA), the polyester block may be designed to include hydrophobic units such as caprolactone units. PLLA or PLGA are more hydrophilic than everolimus, and it is desirable to have a more hydrophobic block of caprolactone so that the polymer would be more hydrophobic to be more miscible with drug.

In some embodiments, the block copolymer comprises at least one polyester block comprising glycolide and a PEG block. The glycolide provides an accelerated or enhanced degradation of the block copolymer. For example, the block copolymer can comprise polyester blocks derived from lactide and glycolide and a PEG block where the glycolide monomer imparts enhanced degradation to the polymer, and the lactide monomer imparts mechanical strength to the block copolymer.

The lactide in the lactide/PEG block copolymer may be D,L-lactide, D-lactide, L-lactide, meso-lactide, or combinations thereof. Such a block copolymer can form a coating with a semi-crystalline morphology where the L-lactide molar concentration can be at least 60% of the polyester block, e.g., more than 80% of the polyester block.

The PEG block also imparts biobeneficial properties to the block copolymer. As used herein, the term "biobeneficial" refers to the attributes of being non-fouling and anti-inflammatory.

In the lactide/PEG block copolymer, the M_{w} of the PEG block generally can range from 1K Daltons to 30K Daltons. However, if is preferred that the molecular weight of the PEG block shall be small enough (e.g., below about 25,000 Daltons) such that the block copolymer can degrade into fragments capable of passing through the kidney membrane.

Some non-limiting examples of the block copolymers are PLGA-PEG-PLGA, P(LA-GA-CL)-PEG-P(LA-GA-CL), P(TMC-GA)-PEG-P(TMC-GA), PLA-PEG-PLA, P(TMC-GA)-PEG-P(TMC-GA), and combinations thereof. As used herein, "LA" is lactide, "GA- is glycolide, "LGA" is lactide-co-glycolide, "CL" is caprolactone, and TMC is trimethylene carbonate.

Some embodiments of the present invention are tri-block polymers formed from lactide, glycolide, and a third monomer that forms a block with a low glass transition temperature, such as without limitation, caprolactone, and trimethylene carbonate. In some embodiments, one block of a tri-block copolymer of the present invention may have a T_{g} below about 60 °C. Ratios of lactide, glycolide and the low T_{g} monomers can vary, forming a tri-block copolymer having different properties, e.g., different degradation rates, different rates of release of a drug from a coating formed of the tri-block copolymer, different drug permeability, different flexibility or mechanical properties. As noted above, generally, the glycolide provides an accelerated or enhanced degradation, and the lactide monomer provides mechanical strength. The third, low T_{g} monomer can enhance drug permeability, water permeability, and enhance the degradation rate of the polymer, imparting greater flexibility and elongation, and improving mechanical properties of a coating formed of the tri-block copolymer.

Monomers such as D-lactide, L-lactide, glycolide, and dioxanone can crystallize if present in high concentration in a polymer. However, crystallization of blocks formed from any of these monomers can be minimized or prevented if concentration of each is below 80% by weight in the polymer. Embodiments of the present invention that are amorphous, or substantially amorphous, tri-block polymers include D-lactide or L-lactide at 10-80% by weight, units of glycolide at 5-80% by weight and units from the third, low T_{g} monomer at 5-60% by weight.

The term "crystalline" refers to having crystallinity of more than 5% in a block copolymer. In some embodiments, the term "crystalline" can refer to having crystallinity of more than 10%, more than 20%, more than 30%, more than 40%, more than 50%, or more than 60% in a block copolymer.

A preferred subset of the block copolymers of the present invention are semi-crystalline diblock and triblock copolymers. The diblock and triblock copolymers have the general formula:

| | |
|---|---|
| A-B | Diblock |
| A-B-A | Triblock |

In the above polymers formulas A represents a polyester block or segment formed from gylcolide and at least one type of lactide monomer, and potentially including other monomers. In the semi-crystalline polymers the lactide may be D-lactide, or L-lactide. Preferably, the molar concentration of these lactide monomers in the A-blocks of the polymer is from 80% to 100%, and more preferably from 82% to 95%. Reference to a mol % in the A block refers to the mol % in all of the A blocks if the polymer has more than one A block. The B block is poly(ethylene glycol). Preferably, the molar concentration in the copolymer of the ethylene glycol monomers is 1 % to 20%, and more preferably 1% to 10%.

The B block may have a weight-average molecular weight (M_{w}) range from 1000 Daltons to 30,000 Daltons, preferably from 1000 Daltons to 20,000 Daltons, and more preferably from 1000 Daltons to 10,000 Daltons. The overall M_{w} of the diblock or triblock polymer is not less than about 50,000 Daltons, preferably not less than 60,000 Daltons, and more preferably, not less than 100,000 Daltons. The overall M_{w} may be not more than 1,000,000 Daltons, and preferably, not more than 600,000 Daltons.

The block copolymers disclosed herein, including the preferred subset, may have various absorption rates. In some embodiments, the block copolymer can have an absorption rate such that about 80% of the mass of the block copolymer is lost in a period of 1 day to 90 days in a physiological environment. In some embodiments, the block copolymer has lost 80% of its mass in a physiological environment in a period from 1 week to 1 year, preferably from weeks to 9 months, and more preferably from 4 weeks to 6 months. Mass loss is due to resorption and/or biodegradation.

Preparation of the block copolymers described herein can be readily accomplished by established methods of polymer synthesis. For example, PLGA-PEG-PLGA can be synthesized by using PEG as an initiator for the ring-opening polymerization of D,L-lactide and glycolide in the presence of stannous octoate as a catalyst.

The block copolymers described herein are useful as coatings on an implantable medical device, or may be used in the fabrication of the device body. The discussion that follows will use a stent as an exemplary implantable medical device, but the embodiments of the present invention are not so limited. The device may be biodegradable and/or resorbable, or biostable. In some embodiments, the implantable device is a biodegradable and/or resorbable stent.

A coating disposed over an implantable device may include a block copolymer described herein in one or more layers in the coating. The coating may be a multi-layer structure. In some embodiments, the coating includes at least one drug reservoir layer, and may include any of the following or any combination thereof:
(1) a primer layer;
(2) a reservoir layer, which can be a drug-polymer layer including at least one polymer (drug-polymer layer) or, alternatively, a polymer-free drug layer;
(3) a topcoat layer, which may be a release rate limiting layer;
(4) a finishing layer.

Embodiments of the present invention also encompass coatings formed by disposing over at least a portion of the outer surface of a device one or more coating formulations, such as, without limitation, disposing one or more coating solutions over the outer surface of the device followed by removal of the solvent. The coating formulations may correspond to any one of the layers described above. The various embodiments referring to a coating of one or more layers also encompass the coating formed by disposing over at least a portion of the outer surface of a device a coating formulation corresponding to each of the one or more layers.

The coating may be disposed over the surface of the device by any number of methods including, but not limited to, electrostatic coating, plasma deposition, dipping, brushing, or spraying. In a preferred embodiment a coating solution is sprayed onto the device. The coating formulation is dissolved, dispersed, and/or suspended in a solvent to form a coating solution. The spraying may be carried out by atomizing the solution and spraying it onto the device surface while rotating and translating the device underneath the spray nozzles followed by rotation and translation under a flow of gas, such as air or nitrogen, which may be heated above room temperature which is 20 °C to 25 °C. Multiple passes underneath the spray nozzles and the gas may be required to obtain a desired layer thickness. Thus, in general, a coating layer is the result of the application of the multiple passes in one process before the device is subjected to an operation for the removal of residual solvent, or before application of a different coating solution. However, in some embodiments the concentration of one substance in the coating formulation, such as the drug, may vary in a layer. Variation throughout the layer may be obtained by application of multiple passes in which the ratio of drug, as a non-limiting example, to other substances is not the same for all of the passes. Materials from one layer may incidentally diffuse or migrate into another layer, or may be extracted by solvent during application of a subsequent layer.

After all layers of the coating have been disposed over the device, or after a particular layer or layers have been disposed over the device, the coating may be optionally annealed at a temperature between 40°C and 150°C, e.g., 80°C, for a period of time between 5 minutes and 60 minutes, if desired, to allow for crystallization of the polymer coating, and to improve the thermodynamic stability of the coating.

The optional primer layer can be disposed over the outer surface of the stent body, and below the reservoir layer to improve the adhesion of the reservoir layer to the stent. The optional topcoat layer can be disposed over at least a portion of the reservoir layer and may serve as a rate-limiting membrane that helps to control the rate of release of the drug. If the topcoat layer is used, the optional finishing coat layer may be disposed over at least a portion of the topcoat layer for further control of the drug-release rate and for improving the biocompatibility of the coating. Without the topcoat layer, the finishing layer may be deposited directly on the reservoir layer.

In some embodiments, the coating may have a drug reservoir layer without any other layers. In other embodiments the coating may have a primer layer or a topcoat layer or both in addition to a drug reservoir layer. In still other embodiments the coating may include all the layers described above. In some embodiments, a coating of the invention may include two or more drug reservoir layers, each of which includes a drug which may the same or different. Additional coating layers not specifically described above may also be included.

The coating can comprise amorphous, or semi-crystalline morphologies. In some embodiments, the coating comprises a semi-crystalline morphology where the block copolymer comprises polyester block having lactide in a molar concentration of at least 80%.

The block copolymers described herein may be used in any layer or layers of the coating in any amount, and may optionally be blended with another biodegradable, resorbable, and/or biocompatible polymer. Non-limiting examples of such polymers are described in U.S. Application Serial No. 12/106,212 filed April 18, 2008.

The coating or coating layers may be disposed over at least a portion of the outer surface of the device body, either directly or indirectly. In some embodiments, the coating or coating layers may be disposed over all of, or substantially all of, the outer surface of the device body. If the coating includes multiple layers, the different layers are not necessarily all disposed over the entire surface, and if not disposed over the entire surface, not necessarily over the same portion of the outer surface. Different types and/or combinations of polymers may be used in different layers. In preferred embodiments, the biodegradable and/or resorbable polymers in a particular layer degrade or are absorbed at a similar or faster rate than those biodegradable and/or resorbable polymers in the layer or layers below. Drug reservoir layers may include more than one drug. It is preferred that the coating layers are not chemically bonded to the surface of the device or to any layer below.

In preferred embodiments, the block copolymer is used to control the release of a drug from a coating. A block copolymer may be combined with a drug in a coating formulation that is disposed over the device to form a drug reservoir layer. For embodiments including the A-B diblock and/or A-B-A triblock copolymers described above, the mass ratio of drug to polymer is preferably less than 1, more preferably about 0.75 or under 0.75, and most preferably about 0.5, or under 0.5.

The coatings including the block copolymers described herein are particularly useful for control of drug release. Embodiments of the present invention including the A-B diblock and/or A-B-A triblock copolymers described above encompass coatings that exhibit a cumulative drug release at 24 hours of not more than 60%, preferably not more than 50%, and more preferably not more than 35%, and/or exhibit a cumulative drug release at 72 hours of not more than 90%, preferably not more than 75%, and more preferably not more than 55%. In some embodiments the coating may exhibit a cumulative drug release at 24 hours of not more than 25%, and/or at 72 hours of not more than 45%.

Some preferred, but not limiting, examples of the drugs that may be included in a coating are paclitaxel, docetaxel, estradiol, 17-beta-estradiol, nitric oxide donors, super oxide dismutases, super oxide dismutases mimics, 4-amino-2,2,6,6-tetramethylpiperidine-1-oxyl (4-amino-TEMPO), rapamycin (sirolimus), Biolimus A9 (Biosensors International, Singapore), deforolimus, AP23572 (Ariad Pharmaceuticals), tacrolimus, temsirolimus, pimecrolimus, novolimus, zotarolimus (ABT-578, Chemical Abstract Services registry number 221877-54-9), 40-O-(2-hydroxy)ethyl-rapamycin (everolimus), 40-O-(3-hydroxypropyl), 40-O-[2-(2-hydroxy)ethoxy]ethyl-rapamycin, 40-O-tetrazolylrapamycin, 40-epi-(N1-tetrazolyl)-rapamycin, dexamethasone, dexamethasone acetate, γ-hiridun, clobetasol, pimecrolimus, imatinib mesylate, midostaurin, feno fibrate, prodrugs thereof, co-drugs thereof, and combinations thereof.

Other preferred bioactive agents include, without limitation, siRNA and/or other oligoneucleotides that inhibit endothelial cell migration. Some further examples of the bioactive agent can also be lysophosphatidic acid (LPA) or sphingosine-1-phosphate (S1P). LPA is a "bioactive" phospholipid able to generate growth factor-like activities in a wide variety of normal and malignant cell types. LPA plays an important role in normal physiological processes such as wound healing, and in vascular tone, vascular integrity, or reproduction.

Other preferred drugs include derivatives of dexamethasone. As used herein, the term "dexamethasone derivatives" encompasses the following specific compounds, without limitation: dexamethasone acetate, dexamethasone palmitate (limethasone); dexamethasone diethylaminoacetate (SOLU-FORTE-CORTIN); dexamethasone isonicotinate; dexamethasone tetrahydrophthalate; and dexamethasone *tert*-butylacetate.

In addition to the preferred drugs and bioactive agents specifically mentioned herein, the implantable medical devices, and/or the coating thereof, may include any of the drugs or bioactive agents listed under the heading "Biologically Active Agents" in U.S. Application Serial No. 12/106,212 filed April 18, 2008.

The foregoing drugs are listed by way of example and are not meant to be limiting. Other biologically active agents that are currently available or that may be developed in the future are equally applicable.

Coatings including the block copolymers described herein exhibit good mechanical integrity, particularly after sterilization. Sterilization of a coated medical device generally involves a process for inactivation of micropathogens. Such processes are well known in the art. A few examples are electron-beam (e-beam), ethylene oxide (ETO) sterilization, and gamma irradiation. Most of these processes involve an elevated temperature. For example, ETO sterilization of a coated stent may involve heating above 50°C at humidity levels reaching up to 100% for periods of a few hours up to 24 hours. Exposure to radiation, such as electron beam, may cause a rise in temperature.

As noted above, the block copolymers described herein are particularly useful when used as part of an implantable medical device, and especially, as part of a coating for an implantable medical device. Coatings including these block copolymers are useful to help control drug release. In addition, it is believed that the use of these block copolymers in coatings may reduce late stage thrombosis. The incidence of late stage thrombosis may be higher for drug-eluting stents as compared to bare metal stents. It is hypothesized that possible causes are the presence of an anti-proliferative drug which potentially may reduce or delay healing, and/or a chronic inflammatory response or hypersensitivity to the polymer in the coating. One means of addressing the potential for hypersensitivity or an inflammatory response to the polymer of the coating is the use of a biodegradable polymer for the coating. However, the biodegradation process may-itself result in inflammation if too rapid. Therefore, it is believed that it is best to use a biodegradable coating that degrades within a year, and preferably within six months. Many of the block copolymers described herein are useful for such coatings.

An additional advantage is that the block copolymers described herein include a resorbable block of poly(ethylene glycol) and at least one biodegradable block. Since part of the copolymer is resorbable, it is believed that coating of such polymers will be absorbed in the blood stream by a dissolving mechanism, thereby mitigating potential side effects caused by small molecule degradation by-products, which may potentially cause inflammation or other adverse reaction in the vessel wall.

The subset of A-B diblock and A-B-A triblock copolymers described herein are particularly useful. Not only do these block copolymers control drug release, they also exhibit acceptable mechanical integrity after sterilization. Moreover, the A blocks or segments are designed to be biodegradable. The B block is designed to be resorbable. As noted above, it is believed that such combination may reduce the potential for inflammation of the vessel wall.

### Methods of Fabricating Implantable Devices

Other embodiments of the invention are drawn to methods of fabricating an implantable device. In one embodiment, the method comprises forming the implantable device of a material including, but not necessarily limited to, a block copolymer as described herein, optionally with one or more other biodegradable, resorbable, or biostable polymers or copolymers, or any combination thereof. Non-limiting examples of such polymers are described in U.S. Application Serial No. 12/106,212 filed April 18, 2008.

In another embodiment, a coating including but not necessarily limited to, the block copolymer described herein may be disposed over the outer surface of a device body resulting in a coating that has a thickness of not more than 30 microns (micrometers), or not more than 20 microns, or not more than 10 microns, or not more than 5 microns.

In some embodiments, a copolymer of this invention optionally including at least one drug may be formed into a polymer construct or preform, such as a tube or sheet that can be rolled or bonded to form a construct such as a tube. A stent may then be fabricated from the tube by cutting a pattern into the tube by laser machining or some other manner. In another embodiment, a polymer construct can be formed from the polymeric material of this invention using an injection-molding apparatus.

### Methods of Treatment

An implantable medical device including a block copolymer as described herein, such as a coated stent or a polymeric stent, may be implanted in a patient to treat medical conditions, such as, without limitation, vascular diseases, or to provide a pro-healing effect.

Medical conditions that may be treated include, without limitation, a vascular disorder such as coronary artery disease (CAD), or peripheral vascular disease (PVD). Some examples of vascular diseases are restenosis and atherosclerosis. Treatment of peripheral vascular disease may include treatment of the superficial femoral artery. Other non-limiting disorders that may be treated include thrombosis, hemorrhage, vascular dissection, vascular perforation, vascular aneurysm, vulnerable plaque, chronic total occlusion, claudication, anastomotic proliferation (for vein and artificial grafts), arteriovenous anastamoses, patent foramen ovale, bile duct obstruction, urethral obstruction, and tumor obstruction. Any combination of the above disorders may be treated with an implantable medical device including a block copolymer as described herein. In particular embodiments, the condition or disorder is atherosclerosis, thrombosis, restenosis or vulnerable plaque.

### EXAMPLES

The embodiments of the present invention will be illustrated by the following examples which are not to be construed as limiting the scope of this invention in any manner.

### Example 1 - Release Rates from Coated Stents

Each of the examples the follows relates to the coating of 3 mm X 12 mm VISION (Abbott Cardiovascular Systems Inc.) stents, which have a coatable surface area of 0.5556 cm². All stents were cleaned by being sonicated in isopropyl alcohol, followed by an argon plasma treatment. No primer layer was applied to the stents. Application of a coating layer on the stents was accomplished by spraying the stents with a 1% acetone solution of everolimus: block copolymer at a mass ratio of 1:1 or 1:2 drug to polymer ratio (D: P).

The spraying operation was carried out with a custom made spray coater equipped with a spray nozzle, a drying nozzle, and a means to rotate and translate the stent under the nozzles with the processing parameters outlined in Table 1. Subsequent to coating, all stents were baked in a forced air convection oven at 50°C for 60 minutes. More than one pass under the spray nozzle was required to obtain the target weight of coating layer on the stent. After heat treatment of the coating, the stents were crimped onto 3.0 X 12 mm XIENCE^{®} V catheters, placed into coil assembly to protect the catheter, and then sealed in Argon filled foil pouches. These stents were sterilized by either electron beam or ethylene oxide sterilization.

**Table 1: Spray Processing Parameters for Coating**

| **Spray Head** | |
|---|---|
| Spray nozzle | .010" ID |
| Spray nozzle temperature, °C | No heat, ambient |
| Atom pres (non-activated), psi | 15 ± 2.5 |
| Spray nozzle to mandrel dist, mm | 11 ± 1 |
| Solution flow rate, ml/hour or ml/min | 0.05+0.03 ml/min |

| **Heat Nozzle** | |
|---|---|
| Temperature at stent site, °C | 62 ± 5 |
| Air Pressure, psi | 20 ± 2 |
| Spray nozzle to mandrel distance, psi | 11 ± 1 |

| **Coating Recipe(s)** | |
|---|---|
| Spray time, seconds | 30 ± 15 |
| Dry time, seconds | 10 |

| **Flow Rate and Coating Weight** | |
|---|---|
| Target Flow Rate (ref.), µg/pass (µg solids per pass) | 18 |

The following PLGA-PEG block copolymers, commercially available from Boehringer Ingelheim, were used in the coating formulations:
A) LGPt8516, an A-B-A triblock copolymer with A blocks of poly(L-lactide-co-glycolide) and B blocks of poly(ethylene glycol), the copolymer having 1 mol% ethylene glycol, and the A block of the copolymer having 85 mol% L-lactide, and the poly(ethylene) B block has a M_{w} of about 6000
B) LGPt8546, an A-B-A triblock copolymer with A blocks of poly(L-lactide-co-glycolide) and B blocks of poly(ethylene glycol), the copolymer having 4 mol% ethylene glycol, and the A block of the copolymer having 85 mol% L-lactide, and the poly(ethylene) B block has a M_{w} of about 6000
C) LGPt8555 an A-B-A triblock copolymer with A blocks of poly(L-lactide-co-glycolide) and B blocks of poly(ethylene glycol), the copolymer having 5 mol% ethylene glycol, and the A block of the copolymer having 85 mol% L-lactide, and the poly(ethylene) B block has a M_{w} of about 5000

The following coating formulations were disposed over the outer surface of the stents:

**Table 2: Summary of Coating Formulations**

| **Lot #** | **Drug: Polymer Mass Ratio** | **Polymer** | **Sterilization Method** |
|---|---|---|---|
| Lot 111307 | 1:1 | LGPt8516 | E-Beam |
| Lot 111307 | 1:1 | LGPt8516 | ETO |
| Lot 111507 | 1:1 | LGPt8546 | E-Beam |
| Lot 111507 | 1:1 | LGPt8546 | ETO |
| Lot 111307 | 1:2 | LGPt8516 | E-Beam |
| Lot 111507 | 1:2 | LGPt8546 | E-Beam |
| Lot 121707 | 1:1 | LGPt8555 | E-Beam |
| Lot 121707 | 1:2 | LGPt8555 | E-Beam |

Cumulative release of the everolimus over 3 days was determined using an United States Pharmacopeia Type VII apparatus (Vankel BIO-DIS^{®} with heat circulation controller). At each time point, 5 stents were removed and saved for drug extraction and drug content analysis. The release testing medium of porcine serum solutions were discarded. The following parameters were employed:
■ Agitation: 40 dpm (dips per minute)
■ Temperature: 37 °C
■ Release Medium: Porcine Serum with 0.1% (w/v) Sodium Azide
■ Time points: day 1, day 3
■ Media volume: 10 ml

The remaining everolimus was extracted from tested stents and analyzed by HPLC. Table 3 summarizes the cumulative release after 1 day and 3 days in porcine serum as a % of the averaged total drug content measured for that particular manufacturing lot. The cumulative release % was calculated based on actual total drug content results. The actual total drug content for each stent was calculated based upon the average percent of drug recovery in the total content assay for the lot times the drug loading per stent based on its coating weight

**Table 3: Cumulative Release Results**

| **Lot #** | **D:P Ratio** | **Polymer** | **Sterilization Method** | **Cumulative Release %** | |
|---|---|---|---|---|---|
| | | | | **1 day** | **3 days** |
| Lot 111307 | 1:1 | LGPt8516 | E-Beam | 92 | 93 |
| Lot 111307 | 1:1 | LGPt8516 | ETO | 97 | 97 |
| Lot 111507 | 1:1 | LGPt8546 | E-Beam | 88 | 98 |
| Lot 111507 | 1:1 | LGPt8546 | ETO | 96 | 97 |
| Lot 111307 | 1:2 | LGPt8516 | E-Beam | 22 | 42 |
| Lot 111507 | 1:2 | LGPt8546 | E-Beam | 5 | 15 |
| Lot 121707 | 1:1 | LGPt8555 | E-Beam | 93 | 94 |
| Lot 121707 | 1:2 | LGPt8555 | E-Beam | 13 | 24 |

### Example 2 - Total Content Assay

Some of the coated stents from Example 1 were also assayed for the total content of the drug. Table 4 summarizes the results of total drug content assay (N = 5 stents) along with the cumulative release results (N = 5 stents) for stents coated with polymer LGPt8516 and everolimus at 100 ug everolimus/cm².

**Table 4: Total Content Assay for Coatings with Polymer LGPt8516**

| **D:P Ratio** | **Sterilization Method** | **Total Content (%)** | **Cumulative Release %** | |
|---|---|---|---|---|
| | | | **1 day** | **3 days** |
| 1:1 | E-Beam | 90.4 ± 1.3 | 92.2 ± 0.4 | 93.4 ± 0.4 |
| 1:1 | EtO | 94.5 ± 1.7 | 96.8 ± 0.3 | 97.1 ± 0.21 |
| 1:2 | E-Beam | 89.3 ± 2.9 | 22.3 ± 5.4 | 42.3 ± 10.4 |

As shown in Table 4 above, for a drug to polymer ratio of 1:1 in the coating obtained using the above spray conditions and solvents, the cumulative release at 1 day is essentially all of the drug in the stent.

### Example 3 - SEM Images

Some of the coated stents of Example 1 that had been sterilized were also subjected to a simulated use test. The simulated use test involves expanding the crimped stents using a catheter balloon pressurized to 16 atmospheres in a simulated lesion made of poly(vinyl alcohol). The catheter balloon pressure was held at 16 atmospheres for 1 minute, after which the balloon was deflated and the catheter retracted to withdraw the balloon. Then deionized water at 37 °C is pumped through the expanded stents at a flow rate of 50 ml/hour for 1 hour. Subsequent to the simulated use protocol, the coating on the stents was analyzed with scanning electron microscopy (SEM). The SEM photographs illustrated that the coatings exhibited acceptable appearance after the simulated use test indicating acceptable mechanical integrity.

## Claims

1. An implantable medical device comprising:
a device body;
a coating disposed over at least a portion of the outer surface of the device body, at least one layer of the coating comprising;
a polymer selected from the group consisting of a semi-crystalline A-B block copolymer, and a semi-crystalline A-B-A block copolymer:
wherein B is a poly(ethylene glycol) block with a weight average molecular weight of 1000 to 30000 Daltons, and A is formed from monomers comprising glycolide, and one or more monomers selected from the group consisting of L-lactide, D-lactide, meso-lactide, and combinations thereof;
wherein the molar concentration of ethylene glycol in the polymer is 1% to 20% and the molar concentration of the sum of L-lactide, D-lactide, and meso-lactide in the A block is 70% to 95%; and
wherein the weight average molecular weight of the polymer is not less than 50,000 Daltons and not more than 1,000,000 Daltons;
and
a drug;
wherein the mass ratio of drug to polymer is less than 1; and
wherein any of the average molecular weight of poly(ethylene glycol) block, the molar concentration of ethylene glycol, and the molar concentration of the sum of L-lactide, D-lactide, and meso-lactide in the A block vary by as much as ±15%.

2. The device of claim 1, wherein the B block of the A-B block copolymer or A-B-A block copolymer has a weight average molecular weight of 1000 to 20000 Daltons.

3. The device of claim 1, wherein the molar concentration of ethylene glycol is 1% to 10% in the A-B block copolymer or the A-B-A block copolymer.

4. The device of claim 1, wherein the molar concentration of the sum of L-lactide, D-lactide, and meso-lactide in the A block is 82% to 95%.

5. The device of claim 1, wherein the device is a stent.

6. The device of claim 5, wherein the stent is biodegradable, resorbable, or a combination thereof.

7. The device of claim 6, wherein the stent body comprises poly(L-lactide).

8. The device of claim 1, wherein the drug is selected from the group consisting of paclitaxel, docetaxel, estradiol, 17-beta-estradiol, nitric oxide donors, super oxide dismutases, super oxide dismutases mimics, 4-amino-2,2,6,6-tetramethylpiperidine-1-oxyl (4-amino-TEMPO), rapamycin (sirolimus), Biolimus A9, deforolimus, AP23572, tacrolimus, temsirolimus, pimecrolimus, novolimus, zotarolimus (ABT-578), 40-O-(2-hydroxy)ethyl-rapamycin (everolimus), 40-O-(3-hydroxypropyl), 40-O-[2-(2-hydroxy)ethoxy]ethyl-rapamycin, 40-O-tetrazolylrapamycin, 40-epi-(N1-tetrazolyl)-rapamycin, dexamethasone, dexamethasone acetate, dexamethasone derivatives, γ-hiridun, clobetasol, pimecrolimus, imatinib mesylate, midostaurin, feno fibrate, and any combination thereof.

9. The device of claim 8, wherein the drug is selected from the group consisting of rapamycin (sirolimus), Biolimus A9, deforolimus, AP23572 , tacrolimus, temsirolimus, pimecrolimus, novolimus, zotarolimus (ABT-578), 40-O-(2-hydroxy)ethyl-rapamycin (everolimus), 40-O-(3-hydroxypropyl), 40-O-[2-(2-hydroxy)ethoxy]ethyl-rapamycin, 40-O-tetrazolylrapamycin, 40-epi-(N1-tetrazolyl)-rapamycin, dexamethasone, dexamethasone acetate, dexamethasone derivatives, and any combination thereof.

10. The device of claim 9, wherein the drug is everolimus, zotarolimus, or a combination thereof.

11. The device of claim 1, wherein the polymer is an A-B-A block copolymer.

12. The device of claim 11, wherein the polymer is selected from the group consisting of a polymer having 85 mol % L-lactide, D-lactide, or a combination thereof in the A-block where the L-lactide and/or D-lactide are among the monomers used in forming the A block, and 1 mol% poly(ethylene glycol) in the polymer where the B block is polyethylene glycol with a weight average molecular weight of 6000, a polymer having 85 mol % L-lactide, D-lactide, or combination thereof in the A block where the L-lactide and/or D-lactide are among the monomers used in forming the A block, and 4 mol% ethylene glycol in the polymer where the B block is polyethylene glycol with a weight average molecular weight of 6000, and a polymer having 85 mol % L-lactide, D-lactide, or a combination thereof in the A-block where the L-lactide, and/or D-lactide are among the monomers used in forming the A block, and 5 mol% ethylene glycol in the polymer, where the B block is polyethylene glycol with a weight average molecular weight of 5000.

13. The device of claim 1, wherein the drug to polymer ratio is 0.75 or less than 0.75, or 0.5 or less than 0.5, wherein the drug to polymer ratio varies by as much as ±15%.

14. The device of claim 1, wherein the device exhibits a cumulative drug release at 24 hours of not greater than 60%, or a cumulative drug release at 72 hours of not greater than 90%, or a cumulative drug release at 72 hours of not greater than 75%.

15. An implantable device as defined in claim 1 for use in the treatment of a disease or condition selected from the group consisting of restenosis, atherosclerosis, thrombosis, hemorrhage, vascular dissection or perforation, vascular aneurysm, vulnerable plaque, chronic total occlusion, claudication, anastomotic proliferation (for vein and artificial grafts), bile duct obstruction, urethral obstruction, tumor obstruction, coronary artery disease (CAD), peripheral vascular disease (PVD), and combinations of these.

## Patentansprüche

1. Implantierbare medizinische Vorrichtung umfassend:
einen Vorrichtungskörper;
eine Beschichtung, die mindestens auf einem Teil der äußeren Oberfläche des Vorrichtungskörpers aufgetragen ist, wobei mindestens eine Schicht der Beschichtung folgendes umfasst:
ein Polymer, das ausgewählt ist aus der Gruppe bestehend aus einem teilkristallinen A-B Blockpolymer und einem teilkristallinen A-B-A Blockpolymer;
wobei B ein Polyethylenglycol-Block mit einem Gewichtsmittel des Molekulargewichts von 1000 bis 30000 Dalton ist, und A aus Monomeren besteht, die Glycolid umfassen und aus einem oder mehreren Monomeren besteht, die ausgewählt sind aus der Gruppe bestehend aus L-Lactid, D-Lactid, Meso-Lactid und Kombinationen davon;
wobei die molare Konzentration von Ethylenglycol im Polymer von 1% bis 20% reicht und die gesamte molare Konzentration von L-Lactid, D-Lactid und Meso-Lactid im A-Block von 70% bis 95% reicht; und
wobei das Gewichtsmittel des Molekulargewichts des Polymers nicht geringer als 50.000 Dalton und nicht höher als 1.000.000 Dalton ist;
und
ein Arzneimittel;
wobei das Gewichtsverhältnis vom Arzneimittel zum Polymer geringer als 1 ist; und wobei das Gewichtsmittel des Molekulargewichts vom Polyethylenglycol-Block und/oder die molare Konzentration von Ethylenglycol und/oder die gesamte molare Konzentration von L-Lactid, D-Lactid und Meso-Lactid im A-Block bis zu ± 15% variieren.

2. Vorrichtung nach Anspruch 1, wobei der B-Block des A-B-Blockcopolymers oder des A-B-A-Blockcopolymers ein Gewichtsmittel des Molekulargewichts von 1000 bis 20000 Dalton hat.

3. Vorrichtung nach Anspruch 1, wobei die molare Konzentration von Ethylenglycol im A-B-Blockcopolymer oder im A-B-A-Blockcopolymer von 1 % bis 10% reicht.

4. Vorrichtung nach Anspruch 1, wobei die gesamte molare Konzentration von L-Lactid, D-Lactid und Meso-Lactid im A-Block von 82% bis 95% reicht.

5. Vorrichtung nach Anspruch 1, wobei die Vorrichtung ein Stent ist.

6. Vorrichtung nach Anspruch 5, wobei der Stent biologisch abbaubar, resorbierbar oder eine Kombination davon ist.

7. Vorrichtung nach Anspruch 6, wobei der Stentkörper Poly(L-Lactid) umfasst.

8. Vorrichtung nach Anspruch 1, wobei das Arzneimittel ausgewählt ist aus der Gruppe bestehend aus Paclitaxel, Docetaxel, Estradiol, 17-Beta-Estradiol, Stickstoffmonoxid-Donatoren, Superoxiddismutasen, Superoxiddismutase-Mimetika, 4-Amino-2,2,6,6-tetramethylpiperidin-1-oxyl (4-Amino-TEMPO), Rapamycin (Sirolimus), Biolimus A9, Deforolimus, AP23572, Tacrolimus, Temsirolimus, Pimecrolimus, Novolimus, Zotarolimus (ABT-578), 40-O-(2-Hydroxy)ethyl-rapamycin (Everolimus), 40-O-(3-Hydroxypropyl), 40-O-[2-(2-Hydroxy)ethoxy]ethyl-rapamycin, 40-O-Tetrazolylrapamycin, 40-Epi-(N1-tetrazolyl)-rapamycin, Dexamethason, Dexamethasonacetat, Dexamethasonderivaten, γ-Hididun, Clobetasol, Pimecrolimus, Imatinibmesylat, Midostaurin, Fenofibrat und einer Kombination davon.

9. Vorrichtung nach Anspruch 8, wobei das Arzneimittel ausgewählt ist aus der Gruppe bestehend aus Rapamycin (Sirolimus), Biolimus A9, Deforlimus, AP23572, Tacrolimus, Temsirolimus, Pimecrolimus, Novolimus, Zotarolimus (ABT-578), 40-O-(2-Hydroxy)ethyl-rapamycin (Everolimus), 40-O-(3-Hydroxypropyl), 40-O-[2-(2-Hydroxy)ethoxy]ethyl-rapamycin, 40-O-Tetrazolylrapamycin, 40-Epi-(N1-tetrazolyl)-rapamycin, Dexamethason, Dexamethasonacetat, Dexamethasonderivaten und einer Kombination davon.

10. Vorrichtung nach Anspruch 9, wobei das Arzneimittel Everolimus, Zotarolimus oder eine Kombination davon ist.

11. Vorrichtung nach Anspruch 1, wobei das Polymer ein A-B-A-Blockcopolymer ist.

12. Vorrichtung nach Anspruch 11, wobei das Polymer ausgewählt ist aus der Gruppe bestehend aus einem Polymer mit 85 Mol.-% L-Lactid, D-Lactid oder einer Kombination davon im A-Block, wobei das L-Lactid und/oder das D-Lactid unter den Monomeren sind, die bei der Bildung des A-Blocks verwendet werden, und 1 Mol.-% Polyethylenglycol im Polymer vorhanden ist, wobei der B-Block Polyethylenglycol mit einem Gewichtsmittel des Molekulargewichsts von 6000 ist, einem Polymer mit 85 Mol.-% L-Lactid, D-Lactid oder einer Kombination davon im A-Block, wobei das L-Lactid und/oder das D-Lactid unter den Monomeren sind, die bei der Bildung des A-Blocks verwendet werden, und 4 Mol.-% Ethylenglycol im Polymer vorhanden sind, wobei der B-Block Polyethylenglycol mit einem Gewichtsmittel des Molekulargewichts von 6000 ist, und einem Polymer mit 85 Mol.-% L-Lactid, D-Lactid oder einer Kombination davon im A-Block, wobei das L-Lactid und/oder das D-Lactid unter den Monomeren sind, die bei der Bildung des A-Blocks verwendet werden und 5 Mol.-% Ethylenglycol im Polymer vorhanden sind, wobei der B-Block Polyethylenglycol mit einem Gewichtsmittel des Molekulargewicht von 5000 ist.

13. Vorrichtung nach Anspruch 1, wobei das Verhältnis vom Arzneimittel zum Polymer 0,75 oder geringer als 0,75 ist, oder 0,5 oder geringer als 0,5 ist, wobei das Verhältnis vom Arzneimittel zum Polymer bis zu ± 15% variiert.

14. Vorrichtung nach Anspruch 1, wobei die Vorrichtung eine kumulative Wirkstofffreisetzung über 24 Stunden aufweist, die nicht größer als 60% ist, oder eine kumulative Wirkstofffreisetzung über 72 Stunden aufweist, die nicht größer als 90% ist, oder eine kumulative Wirkstofffreisetzung über 72 Stunden aufweist, die nicht größer als 75% ist.

15. Implantierbare Vorrichtung wie im Anspruch 1 definiert zur Verwendung bei der Behandlung einer Krankheit oder Störung, die ausgewählt ist aus der Gruppe bestehend aus Restenose, Arteriosklerose, Thrombose, Hämorrhagien, Gefäßdissektion oder Gefäßperforation, Gefässßaneurysma, vulnerablen Plaques, chronischer Totalokklusion, Claudicatio, anastomothischer Profliferation (für Venentransplantate und künstliche Transplantate), Gallengänge-Obstruktion, Obstruktion des Ureters, Tumor Obstruktion, koronarer Herzkrankheit (KHK), peripherer arterieller Verschlusskrankheit (PVD) und Kombinationen davon.

## Revendications

1. Dispositif médical implantable comprenant:
un corps de dispositif;
un revêtement disposé sur au moins une partie de la surface extérieure du corps de dispositif, comprenant au moins une couche du revêtement:
un polymère choisi dans le groupe constitué d'un copolymère séquencé A-B semi-cristalline, et un copolymère séquencé A-B-A semi-cristalline;
dans lequel B est un bloc de polyéthylène glycol avec un poids moléculaire moyen en poids de 1000 à 30000 Dalton et A est formé de monomères comprenant du glycolide, et un ou plusieurs monomères choisis dans le groupe constitué du L-lactide, du D-lactide, du méso-lactide, et des combinaisons de ceux-ci;
dans lequel la concentration molaire de l'ethylène glycol dans le polymère est de 1% à 20% et la concentration molaire du total de L-lactide, D-lactide, et méso-lactide dans le bloc A est de 70% à 95%; et
dans lequel le poids moléculaire moyen en poids du polymère n'est pas inférieur à 50.000 Dalton et n'est pas supérieur à 1.000.000 Dalton;
et
un médicament;
dans lequel le rapport en poids du médicament au polymère est inférieur à 1; et dans lequel l'un quelconque parmi le poids moléculaire moyen du bloc de polyéthylène glycol, la concentration molaire de l'éthylène glycol, et la concentration molaire du total de L-lactide, D-lactide, et méso-lactide dans le bloc A varie jusqu'à ± 15%.

2. Le dispositif de la revendication 1, dans lequel le bloc B du copolymère séquencé A-B ou du copolymère séquencé A-B-A a un poids moléculaire moyen en poids de 1000 à 20000 Dalton.

3. Le dispositif de la revendication 1, dans lequel la concentration molaire de l'éthylène glycol est de 1% à 10% dans le copolymère séquencé A-B ou dans le copolymère séquencé A-B-A.

4. Le dispositif de la revendication 1, dans lequel la concentration molaire du total de L-lactide, D-lactide, et méso-lactide dans le bloc A est de 82% à 95%.

5. Le dispositif de la revendication 1, dans lequel le dispositif est une endoprothèse vasculaire.

6. Le dispositif de la revendication 5, dans lequel l'endoprothèse vasculaire est biodégradable, résorbable, ou une combinaison de ceux-ci.

7. Le dispositif de la revendication 6, dans lequel le corps de l'endoprothèse vasculaire comprend du poly(L-lactide).

8. Le dispositif de la revendication 1, dans lequel le médicament est choisi dans le groupe constitué du paclitaxel, du docétaxel, de l'éstradiol, du 17-béta-éstradiol, des donneurs de monoxyde d'azote, des superoxyde dismutases, des mimétiques de superoxyde dismutase, du 4-amino-2,2,6,6-tétraméthylpipéridine-1-oxyl (4-amino-TEMPO), de la rapamycine (du sirolimus), du Biolimus A9, du déforolimus, de l'AP23572, du tacrolimus, du temsirolimus, du pimécrolimus, du novolimus, du zotarolimus (ABT-578), de la 40-O-(2-hydroxy)éthyl-rapamycine (évérolimus), du 40-O-(3-hydroxypropyl), de la 40-O-[2-(2-hydroxy)éthoxy]éthyl-rapamycine, de la 40-O-tétrazolylrapamycine, de la 40-épi-(N1-tétrazolyl)-rapamycine, de la déxaméthasone, de l'acétate de déxaméthasone, des dérivés de la déxaméthasone, du γ-hididun, du clobêtasol, du pimécrolimus, du mésylate d'imatinib, de la midostaurine, du fénofibrate, et des combinaisons quelconques de ceux-ci.

9. Le dispositif de la revendication 8, dans lequel le médicament est choisi dans le groupe constitué de la rapamycine (du sirolimus), du Biolimus A9, du déforolimus, de l'AP23572, du tacrolimus, du temsirolimus, du pimécrolimus, du novolimus, du zotarolimus (ABT-578), de la 40-O-(2-hydroxy)éthyl-rapamycine (évérolimus), du 40-O-(3-hydroxypropyl), de la 40-O-[2-(2-hydroxy)éthoxy]éthyl-rapamycine, de la 40-O-tétrazolylrapamycine, de la 40-épi-(N1-tétrazolyl)-rapamycine, de la déxaméthasone, de l'acétate de déxaméthasone, des dérivés de la déxaméthasone, et des combinaisons quelconques de ceux-ci.

10. Le dispositif de la revendication 9, dans lequel le médicament est l'évérolimus, le zotarolimus, ou une combinaison de ceux-ci.

11. Le dispositif de la revendication 1, dans lequel le polymère est un copolymère séquéncé A-B-A.

12. Le dispositif de la revendication 11, dans lequel le polymère est choisi dans le groupe constitué d'un polymère ayant 85% mol de L-lactide, de D-lactide, ou d'une combinaison de ceux-ci dans le bloc A où le L-lactide et/ou le D-lactide sont parmi les monomères utilisés dans la formation du bloc A, et 1 % mol de polyéthylène glycol dans le polymère où le bloc B est du polyéthylène glycol avec un poids moléculaire moyen en poids de 6000, un polymère ayant 85% mol de L-lactide, de D-lactide, ou d'une combinaison de ceux-ci dans le bloc A où le L-lactide et/ou le D-lactide sont parmi les monomères utilisés dans la formation du bloc A, et 4% mol d'étylène glycol dans le polymère où le bloc B est du polyéthylène glycol avec un poids moléculaire moyen en poids de 6000, et un polymère ayant 85% mol de L-lactide, de D-lactide, ou d'une combinaison de ceux-ci dans le bloc A où le L-lactide, et/ou le D-lactide sont parmi les monomères utilisés dans la formation du bloc A, et 5% mol d'éthylène glycol dans le polymère, où le bloc B est du poyéthylène glycol avec un poids moléculaire moyen en poids de 5000.

13. Le dispositif de la revendication 1, dans lequel le rapport de médicament au polymère est 0,75 ou inférieur à 0,75, ou 0,5 ou inférieur à 0,5, dans lequel le rapport du médicament au polymère varie jusqu'à ± 15%.

14. Le dispositif de la revendication 1, dans lequel le dispositif présent une libération cumulative de médicament sur 24 heures non supérieure à 60%, ou une libération cumulative de médicament sur 72 heures non supérieure à 90%, ou une libération cumulative de médicament sur 72 heures non supérieure à 75%.

15. Le dispositif implantable tel que définie dans la revendication 1 pour l'utilisation dans le traitement d'une maladie ou d'un état choisis du groupe constitué de la resténose, l'athérosclerose, la thrombose, des hémorragies, la dissection ou perforation vasculaire, l'anévrysme vasculaire, la plaque vulnérable, une occlusion totale chronique, la claudication, la prolifération anastomotique (pour greffes veineuses et artificielles), l'obstruction du canal cholédoque, l'obstruction de l'uretère, l'obstruction tumorale, une maladie coronarienne (CAD), maladie vasculaire périphérique (MVP), et des combinaisons de ceux-ci.
